# DEMANDE DE BREVET EUROPEEN

(11) **EP 2 982 976 A1**
(43) Date de publication de la demande: **10.02.2016**
(21) Numéro de dépôt: 15306242.7
(22) Date de dépôt: 30.07.2015
(51) Int. Cl.: G01N 33/00

(54) **CAPSULE DE GAZ À USAGE UNIQUE, NOTAMMENT DESTINÉE À L'ÉTALONNAGE D'UN CAPTEUR DE PRODUIT POLLUANT**

(30) Priorité: 08.08.2014 FR 1457695
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: Mignot, Guillaume, 60260 LAMORLAYE (FR)
(74) Mandataire: Mellul-Bendelac, Sylvie Lisette

(57) **Abrégé**

L'invention concerne une capsule (1), notamment destinée à l'étalonnage d'un capteur de produit polluant, ladite capsule contenant un gaz, dit principal, et un produit contaminant, formant un mélange, ladite capsule étant configurée pour contenir ledit mélange sous pression et forcer une libération de l'intégralité dudit mélange en une seule dose.

## Description

L'invention concerne une capsule à usage unique, notamment destinée à l'étalonnage d'un capteur de produit polluant, en particulier l'étalonnage d'un capteur de la qualité de l'air.

Il a déjà été proposé des centrales domestiques de mesure de la qualité de l'air. Elles sont destinées à être utilisées par des particuliers ou des collectivités pour contrôler l'atmosphère présente dans les locaux concernés. Elles fonctionnent avec des capteurs donnant une mesure de la concentration du polluant dont on cherche à quantifier la présence. Les concentrations en jeu sont généralement faibles, de l'ordre de quelques centaines à quelques milliers de ppm (parties par million) et les capteurs employés doivent donc être particulièrement sensibles.

Il est connu en ce sens des capteurs utilisant une source émettant un faisceau en direction d'un détecteur. Le faisceau est en partie absorbé par le polluant. La concentration en polluant est ainsi déterminée par comparaison entre une mesure de l'intensité du faisceau en absence de polluant et l'intensité du faisceau partiellement absorbé.

De tels détecteurs connaissent une dérive de leur mesure. Autrement dit, pour une même concentration de polluant, la valeur de la concentration délivrée par le capteur varie d'une mesure à l'autre au fur et à mesure du temps. Dans le domaine des capteurs de mesure de la qualité de l'air, les indications données peuvent varier du simple au double pour une même concentration de polluant. Avec une telle incertitude de mesure, on comprend que l'utilité même de la mesure est compromise.

Pour pallier ce phénomène, il est connu d'étalonner ou de calibrer les capteurs de façon périodique. Pour cela, on met le capteur en contact avec une atmosphère dans laquelle la concentration en polluant est connue et contrôlée et on lance une procédure d'étalonnage ou de calibrage au cours de laquelle on configure le capteur pour qu'il délivre à nouveau une mesure conforme à la concentration en cause. Dans des domaines industriels ou d'équipements urbains, il est ainsi connu de calibrer des capteurs de mesure de polluant en utilisant des bombonnes de gaz contenant un mélange d'air et d'une quantité, calibrée, de polluant. Un opérateur transporte la bombonne à proximité du capteur à calibrer, ouvre la bombonne, libère le mélange en direction du capteur à l'aide d'une buse reliée à la bombonne puis referme la bombonne, ceci tout en lançant une procédure de calibration du capteur. Les bombonnes sont de fortes capacités et servent à la calibration de nombreux capteurs.

On comprend que de telles procédures sont peu pratiques à mettre en oeuvre, notamment compte-tenu du poids de la bombonne. Cette solution ne convient donc pas à des utilisations domestiques. En outre, rien ne limite la quantité de mélange libéré, ce qui peut conduire à certains gaspillages.

Une autre solution pour étalonner les capteurs de centrales domestiques de mesure de la qualité de l'air serait de pouvoir compter sur la présence d'une atmosphère ambiante de référence à intervalles réguliers. Par exemple, dans l'hypothèse où, en pleine nuit, la pollution atmosphérique retomberait toujours à un même niveau, on pourrait lancer une étape de calibration de la centrale de mesure à un moment choisi de la nuit. Malheureusement, cette hypothèse est fausse, surtout en milieu urbain où la qualité de l'air est susceptible de varier fortement, même d'une nuit sur l'autre.

L'invention a pour but de pallier les inconvénients précités et propose à cette fin une capsule, notamment destinée à l'étalonnage d'un capteur de produit polluant, ladite capsule contenant un gaz, dit principal, et un produit contaminant, formant un mélange, ladite capsule étant configurée pour contenir ledit mélange sous pression et forcer une libération de l'intégralité dudit mélange en une seule dose.

Autrement dit, l'invention exclue qu'une vidange seulement partielle de la capsule puisse avoir lieu. Encore autrement dit, ladite capsule est à usage unique. On évite de la sorte la manipulation de bombonnes de poids élevé, qui plus est à ouvrir et fermer entre chaque usage. L'invention permet au contraire de disposer d'une façon particulièrement simple et pratique d'emploi de la quantité juste nécessaire de gaz de calibration.

Ledit gaz principal est, par exemple, un mélange de composés gazeux, en particulier de l'air.

Ledit produit contaminant est choisi, par exemple, parmi les polluants atmosphériques, notamment, le dioxyde de carbone, le monoxyde de carbone, un oxyde d'azote, un oxyde de souffre, un ou des composés organiques volatiles, des particules, en particulier de la fumée issue d'une combustion, ou un mélange de ces composés.

L'invention trouvera ses applications, en particulier, dans le calibrage des capteurs de mesure de la concentration d'un polluant dans l'air, notamment dans le calibrage de centrales domestiques de mesure de la qualité de l'air.

Cela étant, plus généralement, elle trouvera ses applications dans tout domaine où l'on est amené à avoir besoin, dans une quantité limitée, d'un mélange calibré d'un produit contaminant dans un gaz principal.

Selon différentes caractéristiques de l'invention, qui pourront être prise ensemble ou séparément :
- la concentration du produit contaminant dans le gaz principal est comprise entre 1 , notamment 100, et 10.000 ppm,
- la pression du mélange dans la capsule est inférieure à 20 bars, voire à 10 bars, voire à 5 bars,
- le volume de la capsule est inférieur à 200 cm3, voire à 100 cm3, voire à 50 cm3, voire à 25 cm3
- ladite capsule est configurée pour permettre le dégagement d'une ouverture de libération dudit mélange par perçage,
- ladite capsule comprend un orifice de libération dudit mélange et un obturateur dudit orifice,
- ledit obturateur est configuré pour être percé,
- ledit obturateur est une membrane,
- ladite capsule est configurée pour permettre une vidange dudit mélange par retrait dudit obturateur,
- ledit obturateur est configuré pour être escamoté à l'intérieur de la capsule,
- ledit obturateur est configuré pour être repositionné dans ledit orifice,
- ledit obturateur est un bouchon,
- la capsule comprend un corps et un couvercle fermant ledit corps,
- ledit couvercle est configuré pour permettre le dégagement de ladite ouverture de libération du mélange par perçage,
- ledit orifice de libération du mélange est situé au niveau dudit couvercle,
- le couvercle est rapporté sur ledit corps,
- ledit corps et/ou ledit couvercle sont formés par emboutissage,
- ledit corps et ledit couvercle sont issus de matière l'un de l'autre,
- ledit corps et ledit couvercle sont formés par soufflage,
- ledit corps et/ou ledit couvercle sont à base d'aluminium,
- ledit corps et/ou ledit couvercle présente une épaisseur de matière comprise entre 0.1 et 1 mm,
- ledit couvercle présente une surface d'interface avec un support de ladite capsule,
- ladite surface d'interface est configurée pour permettre le dégagement de ladite ouverture de libération du mélange par perçage,
- ledit orifice de libération du mélange se trouve au niveau de ladite interface,
- ledit couvercle comprend un col entre ladite surface d'interface et une partie de section élargie,
- ladite surface d'interface est plane,
- ledit corps comprend un fond et une paroi latérale reliant ledit couvercle et ledit fond,
- ledit fond est plat,
- ladite paroi latérale est de section arrondie,
- ladite capsule présente des rayons reliant ladite paroi latérale et ledit couvercle et/ou ladite paroi latérale et ledit fond.

D'autres caractéristiques et avantages de l'invention apparaîtront à la lecture de la description détaillée qui suit pour la compréhension de laquelle on se reportera aux dessins annexés dans lesquels :
- la figure 1 est une vue perspective d'un exemple de capsule selon l'invention,
- la figure 2 est une vue en coupe longitudinale de la capsule de la figure 1 et d'une partie d'une centrale de mesure de la qualité de l'air destinée à accueillir ladite capsule,
- la figure 3 est une vue similaire à la figure 2 illustrant la mise en place de la capsule sur la centrale,
- la figure 4 est une vue similaire à la figure 2 illustrant la capsule en place sur la centrale lors de la libération du mélange qu'elle contient,
- la figure 5 est une vue similaire à la figure 2 illustrant le retrait de la capsule vide de la centrale,
- la figure 6 est une vue en perspective illustrant une variante de réalisation de la capsule conforme à l'invention,
- la figure 7 est une vue en perspective d'un exemple de centrale de mesure avec laquelle la capsule de la figure 1 est destinée à être utilisée, en condition normale d'utilisation,
- la figure 8 est une vue en perspective de la centrale de la figure 7, prête à être étalonnée.

Dans la description qui va suivre, des référence identiques désignent des pièces identiques ou ayant des fonctions similaires.

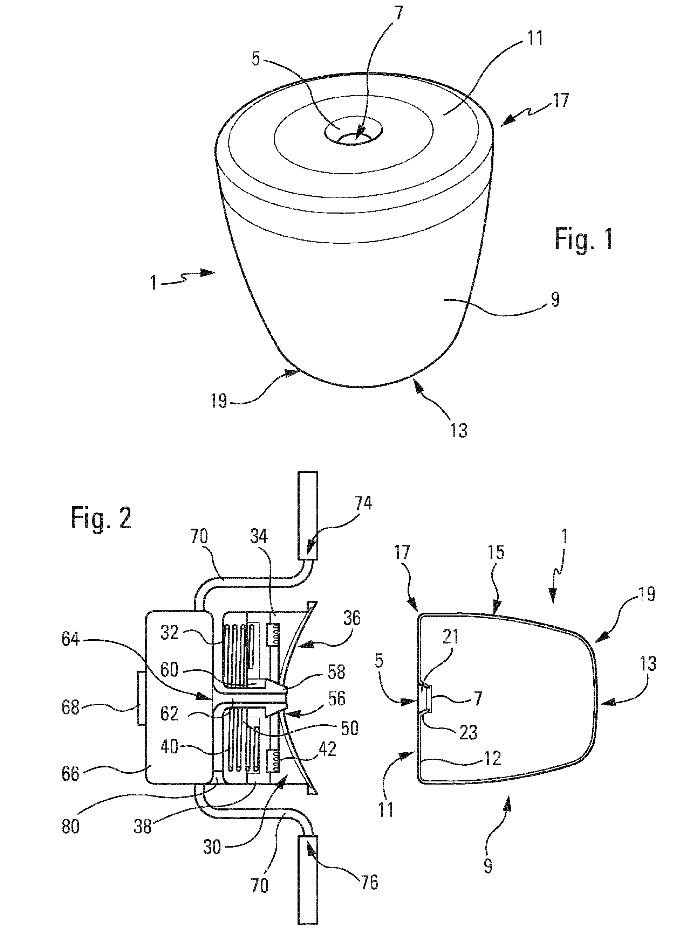

Comme illustré à la figure 1, l'invention concerne une capsule 1, notamment destinée à l'étalonnage d'un capteur de produit polluant.

Ladite capsule contient un gaz, dit principal, et un produit contaminant, formant un mélange. Ledit gaz principal est, par exemple, un mélange de composés gazeux, notamment de l'air. Ledit produit contaminant est choisi, par exemple, parmi les polluants atmosphériques tels que, le dioxyde de carbone, le monoxyde de carbone, un oxyde d'azote, un oxyde de souffre, un ou des composés organiques volatiles, des particules, en particulier de la fumée issue d'une combustion, ou un mélange de ces composés.

Ladite capsule est configurée pour contenir ledit mélange sous pression. On entend par là que ledit mélange est à une pression supérieure à la pression atmosphérique dans ladite capsule de sorte qu'à l'ouverture de ladite capsule, il s'écoulera naturellement vers l'extérieur. Cela étant, une pression excessive n'est pas non plus nécessaire. La pression du mélange dans la capsule pourra ainsi être inférieure à 20 bars, voire à 10 bars, voire à 5 bars. De tels niveaux de pression permettent d'utiliser des capsules dont les coûts de fabrication sont limités.

Ladite capsule est en outre configurée pour forcer une libération de l'intégralité dudit mélange en une seule dose. Autrement dit, un fois la capsule ouverte, elle ne peut plus être refermée. En particulier, ladite capsule ne comprend pas de mécanisme d'ouverture/fermeture, tels que des vannes ou robinets, permettant de la refermer alors que l'intégralité du mélange ne s'est pas encore évacué. Encore autrement dit, ladite capsule est à usage unique.

Elle est de la sorte pratique à manier et à utiliser, notamment en raison de son volume réduit comparé aux bombonnes de gaz connues. A ce sujet, le volume de la capsule est avantageusement inférieur à 200 cm3, voire à 100 cm3, voire à 50, voire à 25 cm3.

La concentration du produit contaminant dans le gaz principal est comprise, par exemple, entre 1 et 10.000 ppm, notamment entre 100 et 10.000 ppm. Pour du dioxyde de carbone, elle pourra être, notamment, d'environ 500 ppm.

A titre d'exemple, le poids de la capsule est de quelques grammes, ce qui illustre bien le côté pratique de son utilisation par opposition aux bouteilles beaucoup plus lourdes utilisées aujourd'hui pour faire de l'étalonnage de capteurs.

Ladite capsule 1 est avantageusement configurée pour permettre le dégagement d'une ouverture de libération dudit mélange par perçage. Ladite capsule 1 comprend ici pour cela un orifice de libération 5 dudit mélange et un obturateur 7 dudit orifice, ledit obturateur 7 étant configuré pour être percé. Il s'agit, par exemple, d'une membrane.

La capsule 1 comprend ici un corps 9 et un couvercle 11 fermant ledit corps 1. C'est avantageusement au niveau du couvercle 11 que le dégagement de l'ouverture de libération du mélange a lieu. Autrement dit, ledit orifice de libération 5 du mélange est situé au niveau dudit couvercle 11, par exemple en son centre.

Selon une première variante, ledit corps 9 et/ou ledit couvercle 11 sont formés par emboutissage, le couvercle 11 étant rapporté sur ledit corps 9, par exemple par sertissage. Selon une autre variante, ledit corps 9 et ledit couvercle 11 sont issus de matière l'un de l'autre. Ils pourront alors être formés par soufflage.

Ledit corps 9 et/ou ledit couvercle 11 sont, notamment, à base d'aluminium. Ils pourront présenter une épaisseur de matière comprise entre 0.1 et 1 mm. Ils pourront aussi être en matériau plastique, notamment en PVC.

Ladite capsule 1, plus particulièrement ledit corps 9, comprend avantageusement un fond 13 plat. Une telle forme facilite le stockage de la capsule et la manipulation de la capsule, notamment lors de son remplissage.

Ladite capsule comprend en outre ici une paroi latérale 15 reliant ledit couvercle et ledit fond 13. Ladite paroi latérale 15 est avantageusement de section arrondie. Ladite capsule pourra en outre présenter des rayons 17, 19 reliant ladite paroi latérale 15 et ledit couvercle 11 et/ou ledit ladite paroi latérale 15 et ledit fond 13. Une telle configuration, en évitant la présence d'arrête vive, facilite la tenue à la pression de la capsule 1.

Ledit couvercle 11 comprend ici un canal 21 de passage du mélange contenu dans la capsule. Ledit canal 21 est formé, par exemple, par un bord plié 23 du couvercle 11, ici tourné vers l'intérieur du capsule. Ladite membrane 7 est fixée au niveau du l'extrémité libre dudit bord plié 23. Plus largement, ladite membrane 7 pourra être fixée à l'extrémité du canal 21 située en regard de l'intérieur de capsule. Ladite membrane 7 est de la sorte située en retrait et protégée des agressions extérieures.

Ledit couvercle 11 est avantageusement plat, ce qui favorisera un arrimage d'un dispositif de vidange de la capsule, en particulier un arrimage étanche. On évite de la sorte les fuites de mélange dans des directions non désirées à l'ouverture de la capsule.

Comme illustré aux figures 2 à 5, un tel dispositif de vidange comprend ici un logement 30 destiné à accueillir ladite capsule 1, en particulier au niveau de son couvercle 11. Ledit logement est, par exemple, de forme sensiblement cylindrique. Ledit logement 30 comprend un fond 32 et une paroi latérale 34, débouchant sur une ouverture 36 d'introduction de la capsule dans ledit logement 30, située ici à l'opposée dudit fond 32 du logement 30.

Ledit dispositif comprend en outre un plateau 38, mobile dans ledit logement 30, et un ressort 40, positionné entre le fond 32 dudit logement 30 et ledit plateau 38. Ledit plateau 38 est ici équipé d'un joint d'étanchéité 42, annulaire, destiné à coopérer avec ladite capsule 1, en particulier son couvercle 11, lorsque ladite capsule 1 est positionnée dans ledit logement 30.

Ledit dispositif comprend en outre un conduit de vidange 50. Ledit conduit 50 passe à travers ledit fond 32 du logement 30 et à travers ledit plateau 38, par des lumières prévues dans ceux-ci, notamment en leur centre.

On comprend que ledit logement 30 est configuré pour positionner, en particulier centrer, ladite capsule par rapport audit conduit de vidange 50, de sorte que celui-ci se trouve en vis-à-vis de la zone de capsule 1 à percer, en particulier de son orifice de libération 5.

Ledit conduit de vidange 50 comprend au niveau de son extrémité distale une connectique 56 en forme d'hameçon, configuré pour percer ledit orifice de libération 5 et s'arrimer à l'intérieur de la capsule contre une face intérieure 12 du couvercle 11. Plus précisément, ici, ladite connectique 56 présente une forme en pointe munie d'un bord postérieur 58 et d'un bord antérieur 60. Un canal 62 du conduit de vidange 50 débouche au niveau dudit bord postérieur 58. Ledit bord antérieur 60 est configuré pour prendre appui contre ladite face intérieure 12 du couvercle, notamment contre l'extrémité libre du bord plié 23.

Ledit conduit de vidange 50 débouche ici à son extrémité proximale 64 au niveau d'une chambre 66 d'accumulation du mélange. Un capteur 68 est situé en regard de ladite chambre 66. Il s'agit, par exemple, d'un capteur permettant de mesurer la concentration du produit polluant en cause. Ladite chambre 66 est reliée à l'extérieur, ici par des canaux 70, 72 débouchant par des évents 74, 76.

La figure 2 illustre plus particulièrement la situation avant mise en place de la capsule 1 dans le logement 30. Le ressort 40 est alors au repos et la connectique 56 est située juste au-delà du plateau mobile 38, vers l'extérieur.

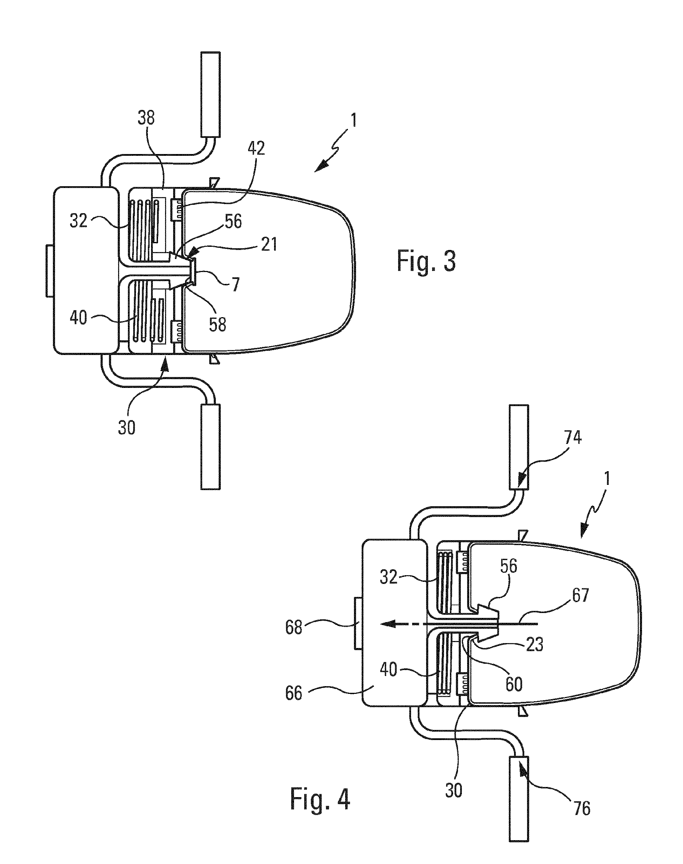

La figure 3 illustre la situation lors de l'introduction de la capsule 1 dans le logement 30. La capsule 1 vient alors en contact avec le plateau mobile 38. Le bord postérieur 58 de la connectique 56 est à ce moment là à l'intérieur du canal de passage 21 de la capsule mais sans avoir encore perforé la membrane 7. La raideur du ressort 40 est avantageusement choisie pour que le joint d'étanchéité 42 soit comprimé par la capsule avant que le plateau mobile ne se déplace vers le fond 32 dudit logement.

La figure 4 illustre la situation après avoir poussé la capsule 1 en direction du fond 32 du logement 30 en lutant contre la force du ressort 40. La connectique 56 a alors percé la membrane 7 et se trouve insérée dans la capsule 1 dans laquelle elle est maintenue par le contact établi entre son bord antérieur 60 et l'extrémité libre du bord plié 23 de ladite capsule 1. La vidange du mélange contenu dans la capsule 1 en direction de ladite chambre d'accumulation 66 a alors lieu, selon la flèche repérée 67, ceci sous l'effet de la différence de pression entre l'intérieur de la capsule et la pression dans ladite chambre d'accumulation 66, qui se trouve à la pression atmosphérique par l'intermédiaire des évents 74, 76. On met de la sorte le capteur 68 en présence d'un mélange dans lequel la quantité de produit contaminant est connu.

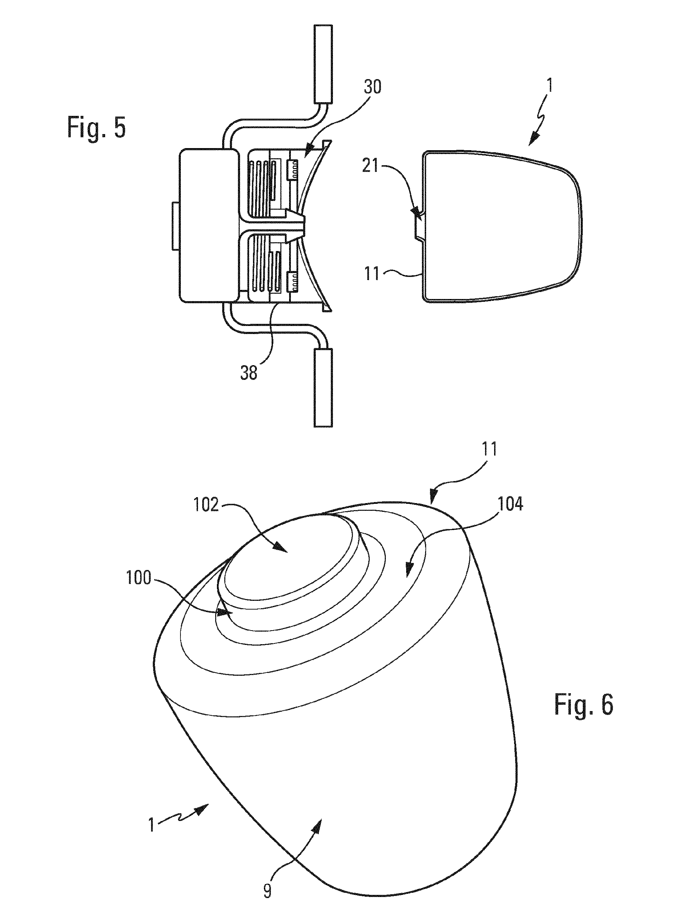

La figure 5 illustre le retrait de la capsule hors du logement 30. Un tel retrait pourra avoir lieu par arrachage de la capsule, l'épaisseur de matière du couvercle 11 étant choisie en ce sens afin de permettre un retournement du canal de passage 21 de ladite capsule. De son côté, le plateau mobile 38 retrouve sa position initiale.

Le remplissage de la capsule 1 pourra avoir eu lieu en usine, par exemple à partir de bouteilles de grandes contenances préparées par voie gravimétrique. On dispose de la sorte dans la capsule d'un produit contaminant dosé de façon très précise car résultant du dosage fait en usine pour le remplissage de la bouteille de grande contenance.

Comme illustré à la figure 6, selon une variante de réalisation de la capsule, ledit couvercle 11 comprend un col 100 entre une surface d'interface 102 et une partie 104 de section élargie. Ladite surface d'interface 102 est ici la partie du couvercle destinée à être introduite dans ledit logement 30 pour coopérer avec ledit plateau mobile 38. En diminuant de la sorte la taille de la partie de la capsule destiné à coopérer avec le dispositif de vidange, on rend plus facile l'utilisation d'une même capsule sur un grand nombre de dispositifs de configuration différente. Ladite surface d'interface 102 pourra présenter un diamètre inférieur à 30 mm, notamment inférieur à 25 mm.

Ladite surface d'interface 102 est configurée pour permettre le dégagement de ladite ouverture de libération du mélange par perçage. Elle pourra en particulier être munie dudit orifice de libération du mélange, non illustré à cette figure. Ladite surface d'interface est avantageusement plane.

Selon un autre mode de réalisation de l'obturateur de la capsule, non illustré, ce dernier pourra être configuré pour être escamoté à l'intérieur de la capsule. Il s'agit, par exemple, d'un bouchon.

Selon cet autre mode de réalisation, ledit obturateur est avantageusement configuré pour être repositionné dans ledit orifice. Plus précisément, ledit bouchon pourra être muni d'une tige. Lorsque ledit bouchon obture ledit orifice de libération du mélange, la tige est configurée pour se trouver intégralement en dehors de la capsule. Lorsque le bouchon a été escamoté à l'intérieur de la capsule, la tige est configurée pour rester au moins partiellement en dehors de la capsule. Son extrémité située à l'extérieur peut de la sorte être manipulée pour repositionner le bouchon à l'intérieur dudit orifice de réception, en tirant sur la tige.

Une telle configuration permet de pouvoir facilement fermer la capsule après un remplissage initial de la capsule.

Elle permet également de pouvoir remplir à nouveau la capsule, après chaque vidange. Cette possibilité technique est permise par cette configuration de fermeture de la capsule, même si on l'a dit plus haut l'esprit de la présente invention est de proposer une capsule à usage unique.

Ladite capsule 1 est plus particulièrement destiné à permette l'étalonnage de capteurs de mesure, tel que le capteur 68 déjà évoqué. En effet, en mettant en contact ledit capteur avec un mélange contenant une quantité prédéfinie de produit contaminant, on peut étalonner ou calibrer ledit capteur autant de fois que désiré.

Ladite capsule 1 est notamment destinée à l'étalonnage de centrales domestiques de mesure de la qualité de l'air, comprenant ledit capteur.

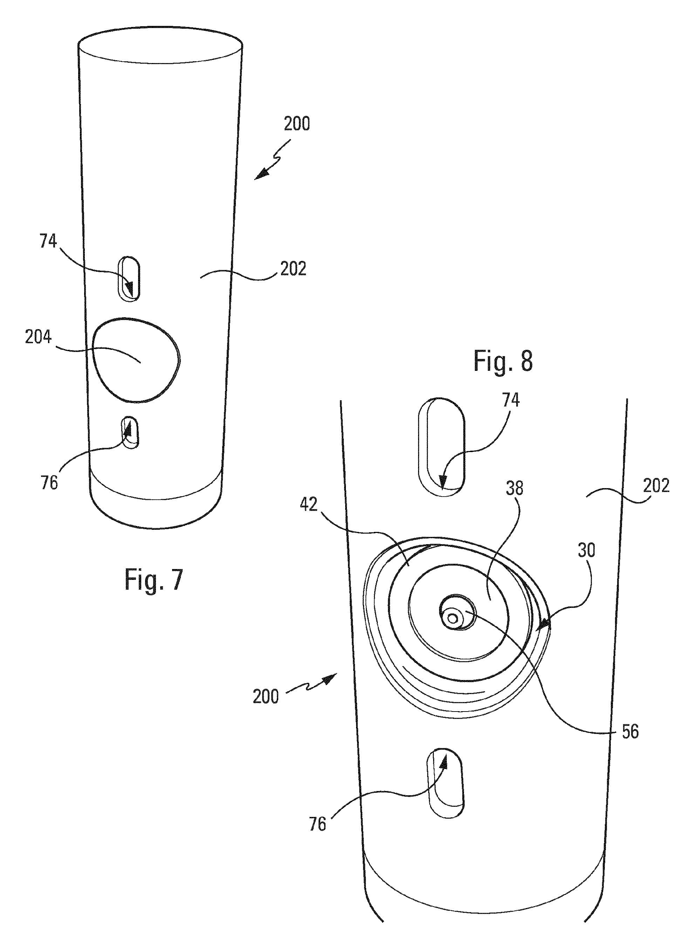

A la figure 7, une centrale 200 conforme à l'invention et destinée à être étalonnée à l'aide de ladite capsule 1 a été illustrée, dans son état de fonctionnement normal, c'est-à-dire, en dehors d'une phase d'étalonnage.

Ladite centrale 200 comprend une chambre d'accumulation de l'air telle que la chambre d'accumulation 66 déjà évoquée, non visible à cette figure. Les évents 74, 76 sont ici prévus au niveau d'un capot 202 de ladite centrale. Ledit capot 202 est, par exemple, de forme sensiblement cylindrique.

Ladite centrale 200 pourra encore comprendre différents organes de traitement, non illustrés, permettant à la centrale d'afficher et/ou de transmettre à distance le résultat de la mesure.

Ledit capot 202 présente ici une forme sensiblement cylindrique définissant une cavité dissimulant ledit capteur, ladite chambre d'accumulation et/ou lesdits moyens de traitement.

A la figure 8, ladite centrale 200 a été préparée pour une phase d'étalonnage. Pour cela une trappe amovible 204 de ladite centrale 200 a été retirée. Elle donne accès à un logement 30 tel que celui du dispositif de vidange décrit plus haut, ledit logement 30 étant ici intégré dans le capot 204. On distingue le plateau mobile 38 et son joint d'étanchéité 42 ainsi que la connectique 56, le reste du dispositif de vidange se trouvant sous le capot 202.

Lesdits organes de traitement de la centrale sont avantageusement configurés pour permettre une phase d'étalonnage de la mesure. Pour cela ladite centrale pourra être munie d'un capteur de détection de la présence d'une capsule 1 dans le logement 30, illustré de façon schématique 80 aux figures 2 à 5. Ladite phase d'étalonnage est ainsi lancée après que ledit capteur ait délivré une information de présence auxdits organes de traitement.

Cela étant, l'invention pourra trouver de nombreuses autres applications. Tout d'abord, ledit capteur de la centrale pourra servir à la détection de plusieurs produits contaminants. Son étalonnage pourra alors se faire à l'aide de la même capsule, contenant chacun des produits contaminant en cause, en quantité contrôlé. Ladite centrale pourra aussi contenir plusieurs capteurs.

Par ailleurs, ladite capsule pourra servir à l'étalonnage de capteur de concentration se trouvant dans tout type de dispositif sans qu'il s'agisse nécessairement de centrales de contrôle de la qualité de l'air, en particulier de centrales domestiques. Il pourra s'agir, par exemple, d'extracteurs, de climatiseurs et/ou de détecteurs de fumée munis de capteurs mesurant la concentration d'un produit contaminant dans l'air ambiant.

Dans de tels cas, le résultat de la mesure effectué par le capteur à calibrer a pour vocation de permettre de contrôler lesdits appareils, notamment en relation avec un seuil de déclenchement.

Ladite capsule pourra encore servir à d'autres applications que des applications d'étalonnage ou de calibrage.

## Revendications

1. Capsule (1), notamment destinée à l'étalonnage d'un capteur de produit polluant, ladite capsule contenant un gaz, dit principal, et un produit contaminant, formant un mélange, ladite capsule étant configurée pour contenir ledit mélange sous pression et forcer une libération de l'intégralité dudit mélange en une seule dose.

2. Capsule (1) selon la revendication 1 dans laquelle ledit gaz principal est de l'air et/ou ledit produit contaminant est choisi parmi le dioxyde de carbone, le monoxyde de carbone, un oxyde d'azote, un oxyde de souffre, un ou des composés organiques volatiles, des particules, en particulier de la fumée issue d'une combustion, ou un mélange de ces composés.

3. Capsule (1) selon l'une quelconques des revendications précédentes dans laquelle la concentration du produit contaminant dans le gaz principal est comprise entre 1 et 10.000 ppm.

4. Capsule (1) selon l'une quelconque des revendications précédentes dans laquelle la pression du mélange dans la capsule est inférieure à 20 bars, voire à 10 bars, voire à 5 bars.

5. Capsule (1) selon l'une quelconque des revendications précédentes dans laquelle le volume de la capsule est inférieur à 200 cm³.

6. Capsule (1) selon l'une quelconque des revendications précédentes dans laquelle ladite capsule est configurée pour permettre le dégagement d'une ouverture de libération dudit mélange par perçage.

7. Capsule (1) selon l'une quelconque des revendications précédentes dans laquelle ladite capsule (1) comprend un orifice de libération (5) dudit mélange et un obturateur (7) dudit orifice (5), ledit obturateur (7) étant configuré pour être percé et/ou escamoté à l'intérieur de la capsule.

8. Capsule (1) selon la revendication 7 dans laquelle ledit obturateur (7) est une membrane.

9. Capsule (1) selon la revendication 7 dans laquelle ledit obturateur (7) est un bouchon.

10. Capsule (1) selon l'une quelconque des revendications 8 ou 9 dans lequel ledit obturateur (7) est configuré pour être repositionné dans ledit orifice.

11. Capsule (1) selon l'une quelconque des revendications précédentes dans lequel la capsule (1) comprend un corps (9) et un couvercle (11) fermant ledit corps, ledit (11) couvercle étant rapporté sur ledit corps (9).

12. Capsule selon la revendication 11 dans laquelle ledit corps (9) et/ou ledit couvercle (11) sont emboutis.

13. Capsule selon la revendication 11 dans laquelle ledit couvercle (11) présente une surface d'interface (102) avec un support de ladite capsule (1), ledit couvercle (11) comprenant un col (100) entre ladite surface d'interface (102) et une partie (104) de section élargie.

14. Capsule selon la revendication 13 dans laquelle ladite surface d'interface (102) est plane.

15. Capsule selon l'une quelconque des revendications 11 à 14 dans laquelle ledit corps (9) comprend un fond (13) et une paroi latérale (15) reliant ledit couvercle (11) et ledit fond (13), ledit fond (13) étant plat, ladite paroi latérale (15) étant de section arrondie et ladite capsule (1) présentant des rayons (17, 19) reliant ladite paroi latérale (15) et ledit couvercle (11) et/ou ladite paroi latérale (15) et ledit fond (13).
